# EUROPEAN PATENT SPECIFICATION

(11) **EP 0 440 710 B1**
(45) Date of publication and mention of the grant of the patent: **11.05.1994**
(21) Application number: 89912194.1
(22) Date of filing: 24.10.1989
(51) Int. Cl.: A61K 31/445, C07D 221/14

(54) **NEW ANTIVIRAL AGENTS**
NEUE ANTIVIRALE MITTEL
NOUVEAUX AGENTS ANTIVIRAUX

(30) Priority: 24.10.1988 US 261436; 11.09.1989 US 405834
(43) Date of publication of application: 14.08.1991
(73) Proprietor: SCRIPPS CLINIC AND RESEARCH FOUNDATION, La Jolla California 92037 (US)
(72) Inventor: RIDEOUT, Darryl, C., San Diego, CA 92126 (US); ELDER, John, Encinitas, CA 92024 (US)
(74) Representative: Goldin, Douglas Michael
(86) International application number: PCT/US89/04773
(87) International publication number: WO 90/04394

(56) References cited:
- FR-A- 2 574 184
- US-A- 4 200 436
- US-A- 4 215 102
- US-A- 4 460 560
- ANALYTICAL BIOCHEMISTRY, vol. 159, no. 1, 1986, pages 179-186, Academic Press, Inc.; B. DESFOSSES et al.: "The use of disulfonatonaphthalimide fluorescent dyes for the fluorescence polarization immunoassay of steroids"
- Journal of Medicinal Chemistry vol.22, No.11. 1979, Neal Springarn et al., "Synthesis and Evaluation of Thiosemicarbazone, Dithicarbozonate, and 2'-Pyrazinylhydrazone of Pyrazinecarbozaldahyde as Agents for the Treatment of Iron Overload",pages 1314-1316
- Journal of Medicinal Chemistry, vol. 25, No 5 1982, K. Murdock et al.,"Antitumor Agent 2. Bisguanyl Hydrazone of Anthracene-9, 10-dicarboxaldehydes"pages 505-518

## Description

The invention relates to materials which are useful in treating or inhibiting viral infection. More specifically, it concerns self-assembling naphthyl disulfonates and also concerns semicarbazones of Lucifer Yellow and its analogs which are effective in inhibiting human immunodeficiency virus (HIV) infection and other retroviral infections.

There are a number of viral diseases recognized as retroviral in origin in both humans and other animals. The most publicized such disease among humans is that caused by the human immunodeficiency virus (HIV) as either AIDS or ARC. Other such diseases, however, include hepatitis B and hepatitis delta. Among cats, retroviral diseases include those caused by the feline immunodeficiency virus (FIV) and the feline leukemia virus (FeLV). A number of other animal species also contract retroviral-caused infections, such as the Visna virus infections of ungulates.

There have been a number of approaches to the treatment of such diseases, none of them entirely successful. Over a million Americans and several million people worldwide are infected with HIV, and treatment with the experimental drug AZT, produced by Burroughs-Wellcome, appears to be of some help although it is clear that AZT is unable to cure AIDS and is extremely toxic. Nevertheless, AZT has been a considerable commercial success. There is no doubt that there is a continuing need for more effective antiviral agents which are effective against retroviral infections in general, and against HIV in particular.

It is recognized that there are many advantages to assembling a biologically active substance at the site of its intended activity, especially when the active substance can be formed from innocuous and nontoxic precursors. PCT Application W088/00047, published 14 January 1988 and incorporated herein by reference, describes various materials which can be administered separately to a microenvironment and there assembled to create an active conjugate. One microenvironment which is of considerable interest is any of the loci of infection by human immunodeficiency virus (HIV), and the possibility to administer nontoxic precursors of a drug which would self-assemble at the site of the HIV infection would clearly be advantageous in treating the disease and symptomology caused by this virus.

While the published PCT application describes generically the concept of active conjugate self-assembly at a desired locus, and provides certain exemplification of this concept, no specific compounds useful in the treatment of HIV infection are disclosed.

In addition, rather than relying on self-assembly at the site of infection, preassembled compounds can be used which are related to those used as components in the assembly. Certain additional art discusses compounds which are also related to those described and claimed herein.

Various active compounds have been suggested for the treatment of HIV infection, the best known being AZT and dextran sulfate. The compound most closely related to the invention conjugates described herein is suramin sodium, which is a complex conjugate of trisodium sulfonate naphthyl derivatives and aromatic nuclei linked through amide linkages. The structure of this compound is: 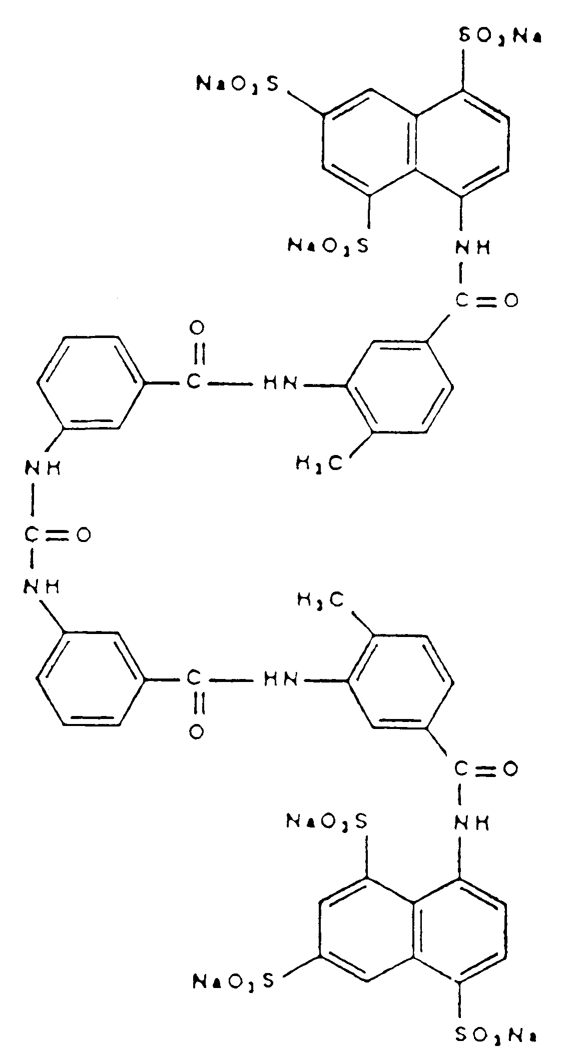
The effects of suramin on the treatment of AIDS and its mechanism of action have been studied by a number of workers (Balzarini, J., et al, Int J Cancer (1986) 37:451-457; Levine, A.M., et al, Annals Int Med (1986) 105:32-37; Broder, S., et al, Lancet (1985) 1:627-630; Cheson, B.D., et al, JAMA (1987) 258:1347-1351). In general, this compound has been found helpful in treatment, although its toxicity precludes its use as a single agent in treatment. Side effects include ophthalmic damage, hypoadrenalism, nausea, thrombocytopenia and vomiting, and some deaths have been attributed to these side effects.

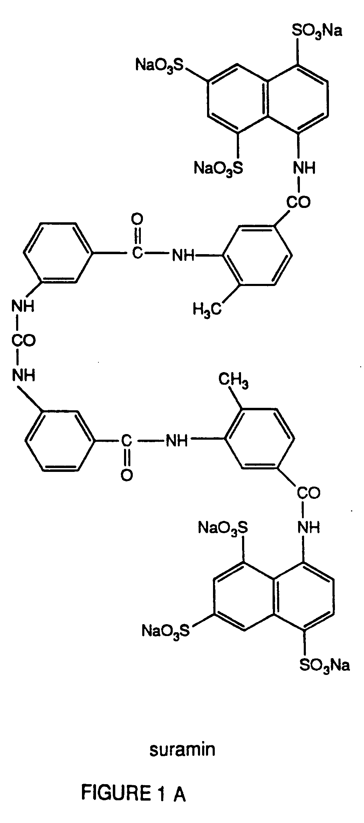

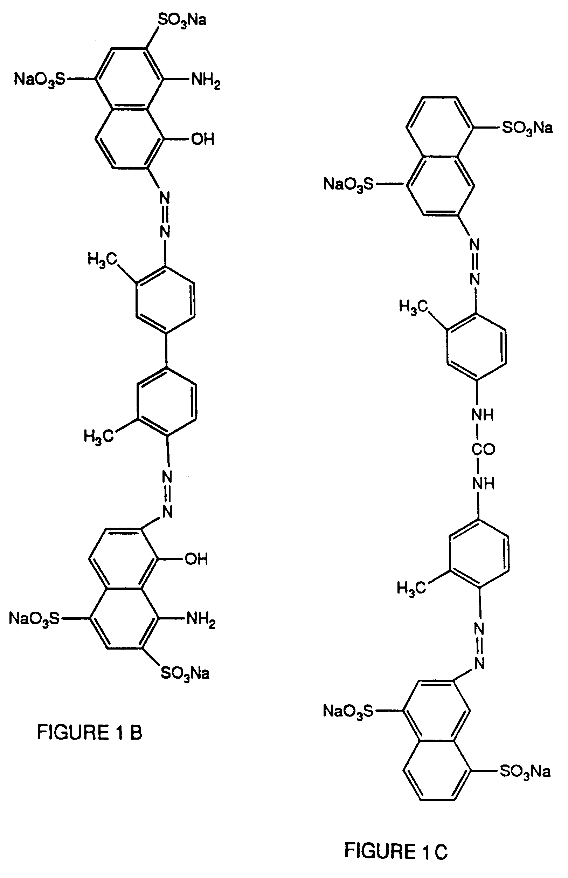

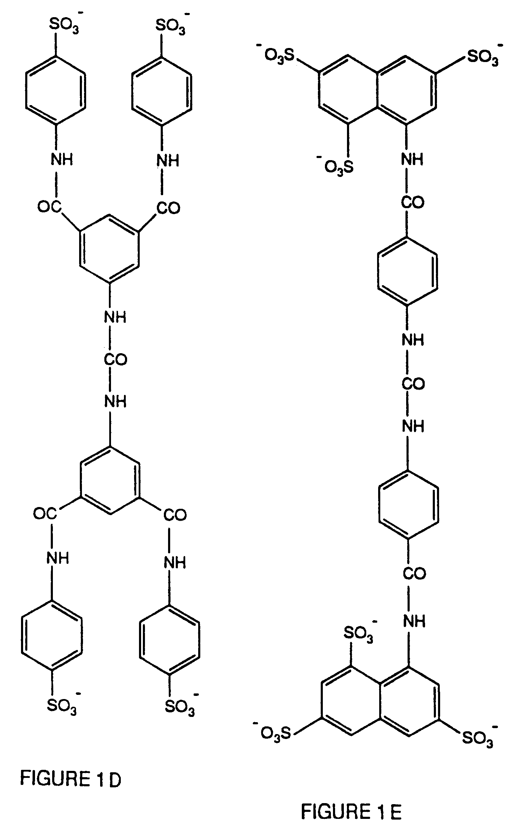

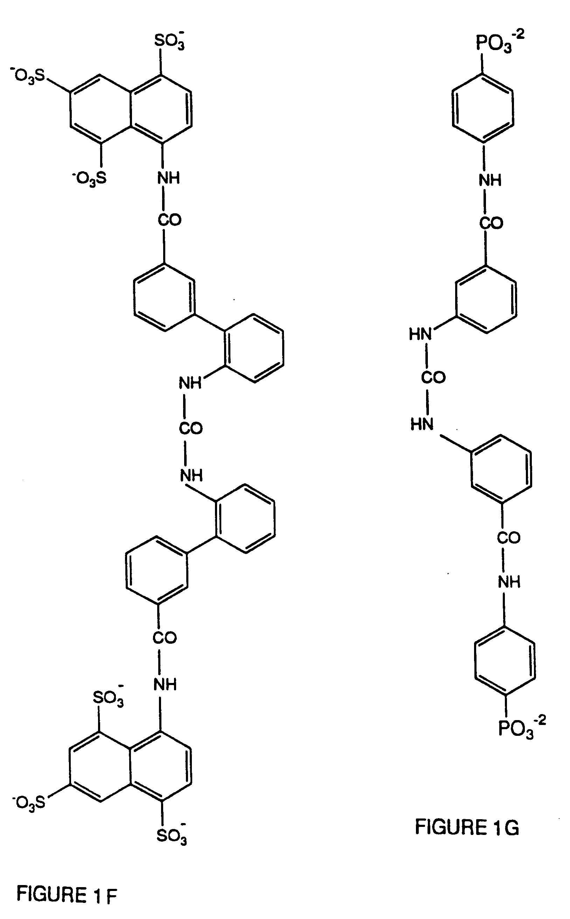

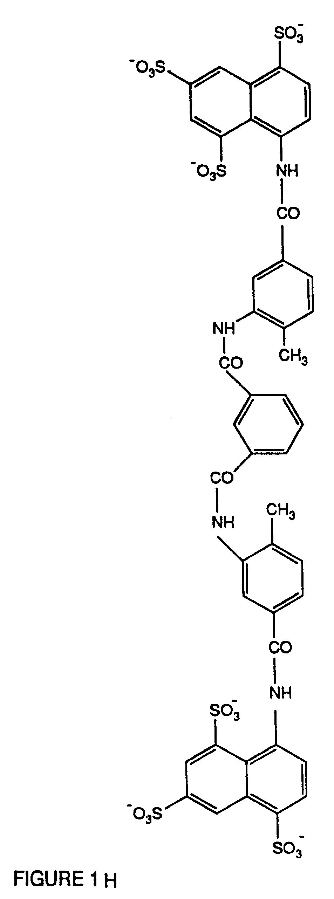

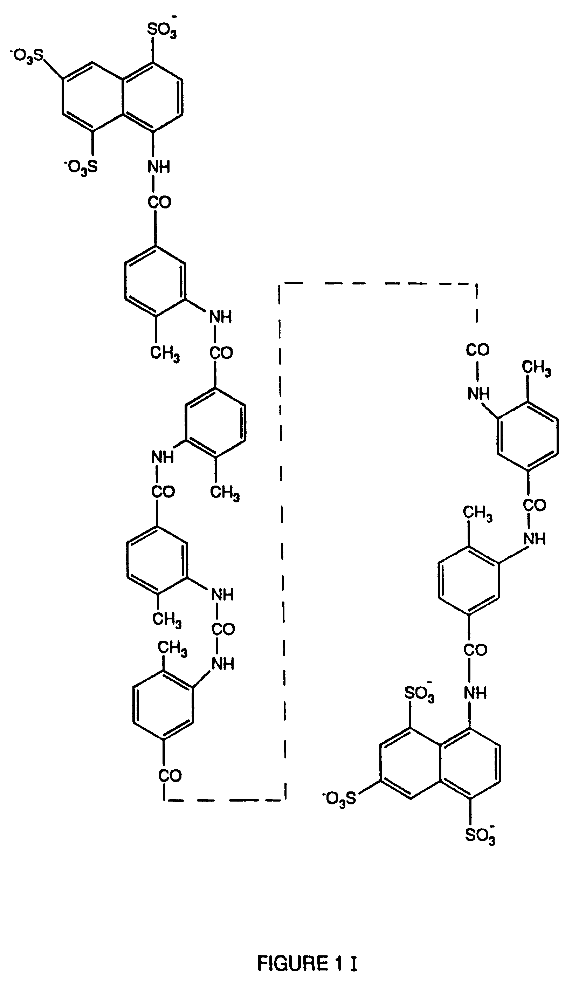

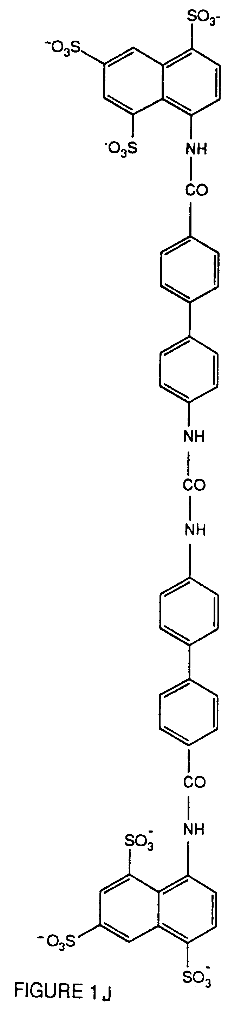

It has been suggested that the antiviral mechanism of suramin is due to binding of the polyanionic molecule to reverse transcriptase (DeClercq, E., Cancer Lett (1979) 8:9-22) and a large number of suramin analogs have been prepared. A total of 90 suramin analogs, of which 57 showed the ability to inhibit HIV-I reverse transcriptase activity, and 24 of which were superior to suramin in this inhibition, were prepared by Jentsch, K.D., et al, J Gen Virol (1987) 68:2183-2192. A representative group of suramin analogs is shown in Figure 1. All of these are bis(naphthalene polysulfonic) acids, show ID₅₀ values in the range of 5-42 ug/ml and, like suramin, eliminate the cytopathic effect of HIV-I against MT4 cells at 50 ug/ml.

As stated above, suramin sodium is highly toxic; too toxic be used clinically to treat HIV infection. The analogs so far prepared have similar drawbacks. Further, neither suramin nor its analogs offer the opportunity for localized formation through in situ self-assembly, as they are conjugated through amide or urea linkages which are not formed readily under in vivo conditions.

The precursors and conjugates of the herein invention appear to home to monocytes, as is described below. It appears that monocytes and macrophages are an appropriate target for an antiviral agent directed to HIV and other retroviral infection. In AIDS patients, evidence of HIV-I infection has been observed in monocytes from a variety of tissues, including brain, peripheral blood, lymph nodes, skin, and lung (Pauza, C.D., et al, J Virol (1988) 62: (in press); Pauza, C.D., Cell Immunol (1988) 112:414-424) and human peripheral blood monocytes and monocyte-derived cell lines support viral replication in vitro. In addition, two other immunosuppressive retroviruses, MLDV and the Visna virus of ungulates, cause immunosuppression primarily by infecting monocytes.

Others have shown that monocytes act as a reservoir for virus in infection (Ho, D., et al, New Eng J Med (1987) 317:278-286; Klatzmann, D., et al, Immunol Today (1986) 7:291-296). Since few T-cells are actually infected with HIV-I in AIDS patients, and since it is known that infected monocytes can shed HIV-I envelope protein in soluble form, it is inferred that infected monocytes may mediate the loss of helper T-cells in these patients (Siliciano, R.F., et al, Cell (1988) 54:561-575).

FR-A- 2574184 discloses compounds containing a fluorescent group which may be linked to an antibody or protein and are useful in immunoassay techniques. The fluorescent group corresponds to a group present in the compounds of the invention.

Some of the compounds of the present invention offer the capability of self-assembly in vivo and offer the capability to interfere with the life cycle of retroviruses in general, probably through interference with the activity of reverse transcriptase, and are thus useful as prophylactic and therapeutic agents in the treatment of retroviral infections.

### Disclosure of the Invention

Nontoxic precursors have been devised for conjugates capable of inhibiting HIV infection in human subjects, as well as relatively nontoxic conjugates capable of inhibiting retroviral infection in human and animal subjects. The precursors are capable of self-assembly after administration selectively at the locus of HIV infection to form conjugates which are toxic to the virus. This permits the treatment of patients with materials which are biologically safe to the subject, which nevertheless become toxic to the target infection when assembled at the appropriate site. Because the assembly is largely restricted to the locus of the target virus, any toxic side effects of the assembled conjugate to the patient being treated are minimized. The conjugates administered per se are also toxic to the retrovirus, and appear to inhibit reverse-transcriptase.

In one aspect, the invention is directed to conjugates obtained from these precursors. These conjugates are derivatives of the commercially available nontoxic dye, Lucifer Yellow CH (LY-CH), and its analogs.

The present invention therefore provides compounds of formula (1)

R-NHCONHN=CH-Y-CH=NNHCONH-R (1)

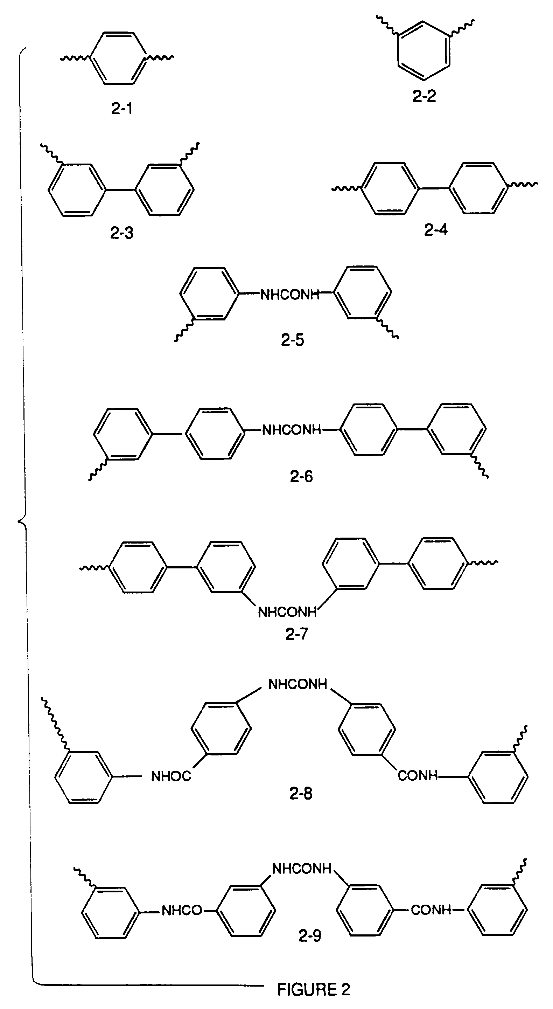

wherein R is of the formula: 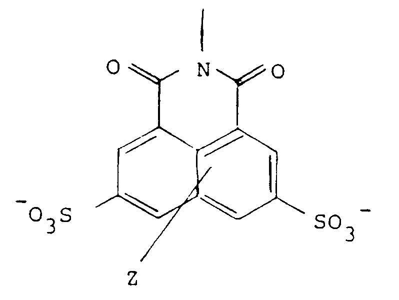
wherein Z represents one or two non-interfering substituents, and
Y is selected from the group consisting of the moieties 2-1 to 2-9 shown in Figure 2.

The conjugates are formed by the in situ reaction of precursors of the formula:

R-NHCONHN=CH-Y-CHO (2)

and

R-NHCONHNH₂ (3)

wherein R and Y are as above defined.

Accordingly, in a further aspect, the invention is directed to a process for producing compounds of formula (1), which comprises contacting a compound of formula (2) and a compound of formula (3).

In another aspect, the invention is directed to pharmaceutical compositions useful in the treatment of HIV infection containing precursors of a compound of formula (1) capable of self-assembly into the compound of formula (1) in the presence of virus-infected cells. In particular it relates to a composition containing, as active ingredients, compounds of formula (2) and formula (3).

In a further aspect, the invention relates to combinations of such precursors, and in particular of compounds of formulae (2) and (3), the use of such precursors in the manufacture of a medicament for use in the treatment of retroviral infection in a human subject, and precursors, which are compounds of formula (2), for such use.

In another aspect, the invention is directed to compounds of formula (4) 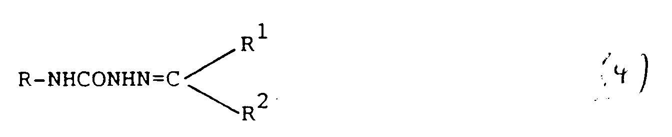
wherein R is wherein R is as defined in relation to formula (1)
R¹ is H or alkyl (1-6C); and
R² is alkyl (1-6C) or aryl (6-10C) or "linked" aryl (6-10C) containing 2-5 such aryl groups, said alkyl or aryl substituents being unsubstituted or substituted by 1 or 2 non-interfering substituents, pharmaceutically acceptable salts thereof and pharmaceutical compositions containing them.

In still another aspect, the invention is directed to a method to prepare the compounds of Formula (4) which comprises contacting a compound of the formula (3) RNHCONHNH₂ with a compound of the formula (5) R¹R²CO, and recovering the resulting compound of formula (4).

### Brief Description of the Drawings

Figure 1 shows representative analogs of the compound suramin sodium. Suramin sodium is shown in this figure as Structure A.

Figure 2 shows embodiments of the moiety Y in the precursors and conjugates of the invention.

Figure 3 shows the synthesis of precursors A1-A9 and conjugates H1-H9.

### Modes of Carrying Out the Invention

### A. Nature of the Precursors and Conjugates

The conjugates formed in situ and the precursors thereof are derived from the nontoxic commercially available dye Lucifer Yellow (LY-CH)or its analogs. LY-CH has the formula: 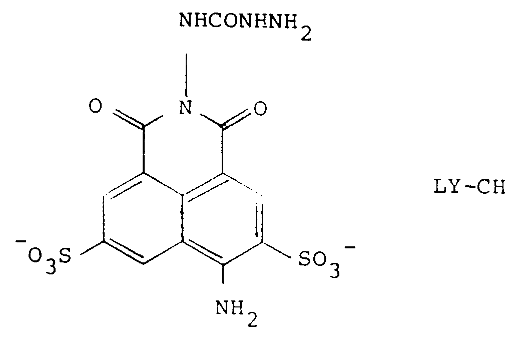
and is thus a semicarbazide. The commercially available material is in the form of the lithium salt; however, other salts of the sulfonates, or even the free acid forms, can be used in the formulation. Of course, the ionization status of the sulfonate will depend on the pH of the medium which surrounds it. Suitable salts for use when the compound is to administered therapeutically include any nontoxic, inorganic ions, such as sodium, potassium, ammonium, calcium, magnesium, and so forth, or may also be organic cations, such as quaternary amines. In general, inorganic ions are preferred, as these confer greater solubility on the compounds and are less expensive. The free acid form can also be used in the manufacture of a suitable formulation which will adjust the pH to a physiologically compatible value.

By "analogs" of the Lucifer Yellow moiety is meant compounds of the formula: 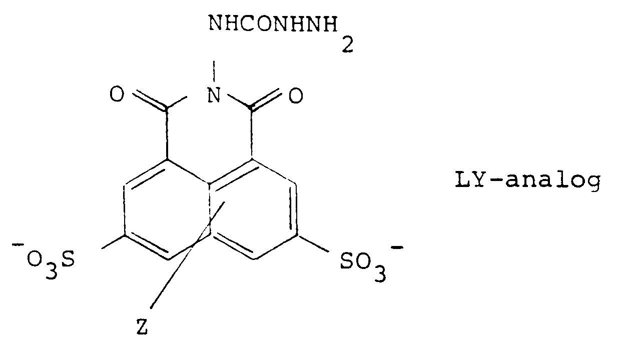
wherein Z represents one or two innocuous substituents in either ring such as lower alkyl, lower alkoxy, hydroxy, amino, thio, alkylamino, alkylthio, or halo. The nature of Z is not important in these analogs so long as it does not interfere with the self-assembly, with the efficacy of the conjugate, or with the non-toxicity of the precursor. Thus, by "analogs" of Lucifer Yellow, is meant those naphthalene disulfonates related to Lucifer Yellow which have different substituents in the naphthalene nucleus other than the single amino group present in Lucifer Yellow itself. As is the case with Lucifer Yellow, the analog sulfonates may be in the form of the free acid or any pharmaceutically acceptable salt.

With respect to the self-assembling hydrazones, calculations based on the known uptake characteristics of LY-CH, the relative volumes of endosomes and lysosomes inside cells, and various kinetic parameters known to describe formation of hydrazones, it can be calculated that antiviral concentrations of the product hydrazone can be obtained inside monocyte lysosomes using sub-cytotoxic concentrations of the precursors, and without forming appreciable conjugate outside of the monocyte. These calculations are done manually or using computer simulation.

In addition, verification of the various parameters for either the self-assembling hydrazones or preferred semicarbazones can be obtained using a model system in cats involving feline immunosuppressive virus (FIV) which is a T-lymphotropic lentivirus, wherein the virus can infect feline T-lymphocytes, feline peripheral monocytes derived macrophages and CCL94 feline kidney-derived fibroblasts in vitro. As the symptoms of FIV are similar to those of AIDS infections in humans, this provides a suitable model system for HIV and for retroviral infection in general (Pederson, N. C., et al, Science (1987) 235:790-793; Elder, J., (1988, personal communication)).

The cytotoxicity of Lucifer Yellow and the analogs within the scope of the invention is low. The LD₄₀ for LY-CH injected intravenously in mice is greater than 1 g/kg (Silverstein, S., (1987, personal communication)) and LY-CH exhibits no observable cytostatic activity in vitro against murine thiol-macrophages at 600 uM concentrations (Swanson, J.A., et al, J Cell Biol (1985) 100:851-859).

Preferred forms of the precursors include those wherein R represents the tricyclic residue of Lucifer Yellow-i.e., Z is NH₂ at the position shown. Preferred embodiments of Y are those represented by formulas 1-4 of Figure 2, and in particular, most preferred is that represented by formula 2-2 of Figure 2. Thus, particularly preferred as precursors are Lucifer Yellow CH itself, in the form of any pharmaceutically acceptable salt, as the compound of formula 3, and the compound designated A2 herein, of the formula: 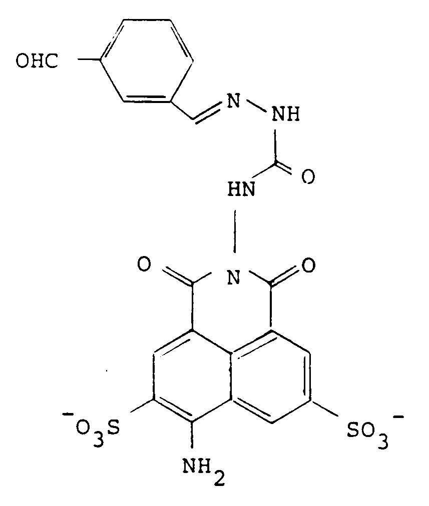
which is a condensation product of LY-CH with the benzene-1,3-dialdehyde, as the compound of formula (2).

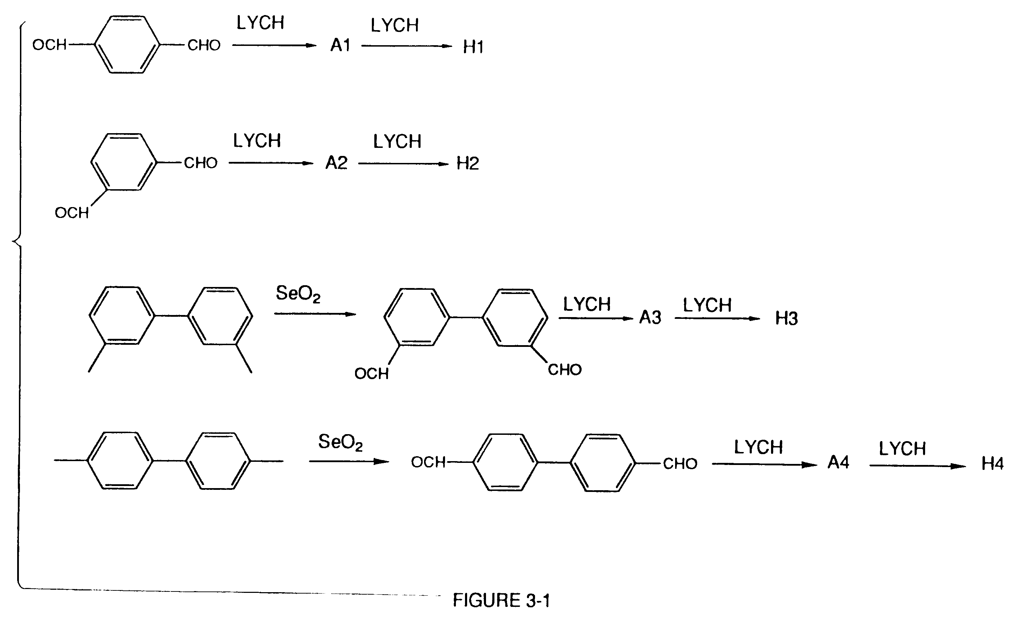

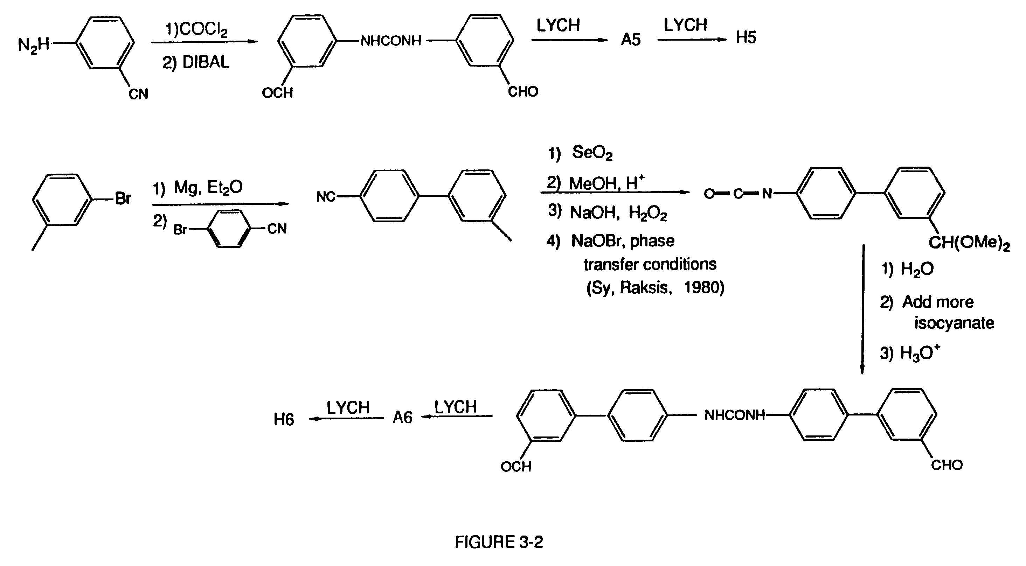

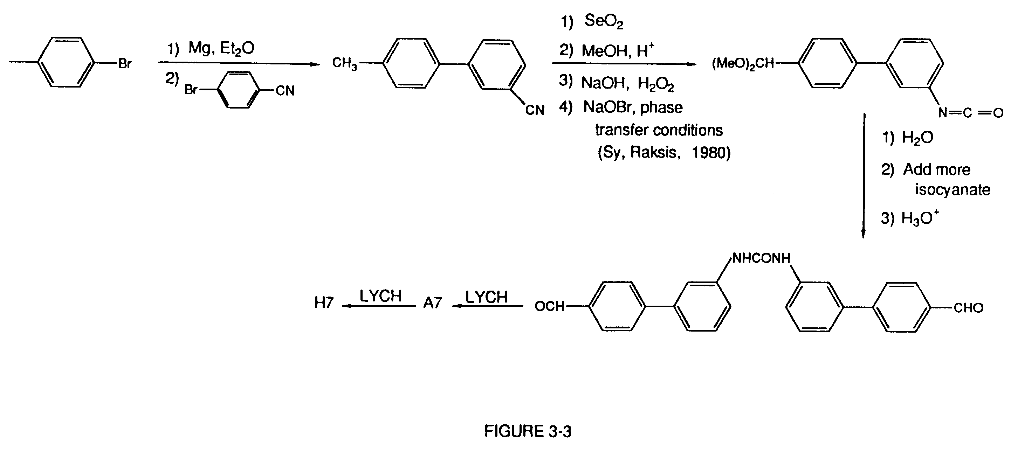

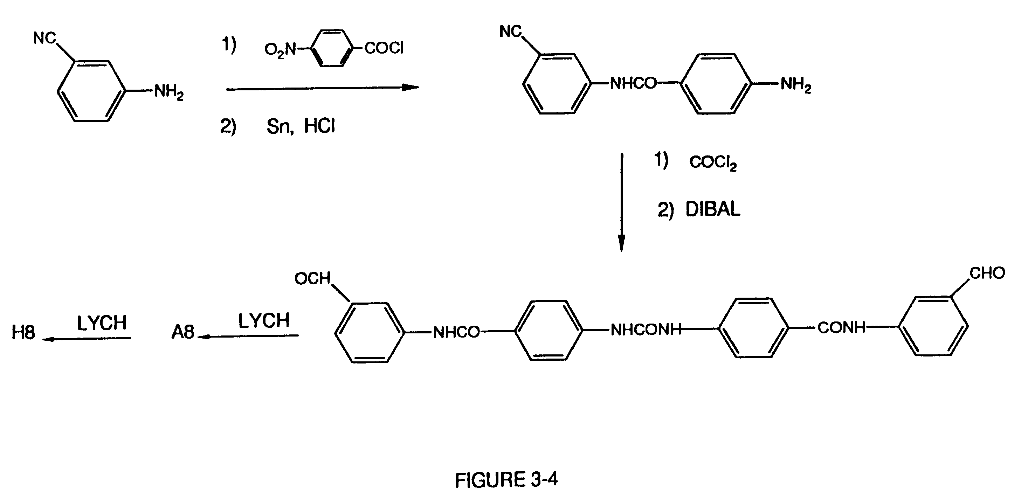

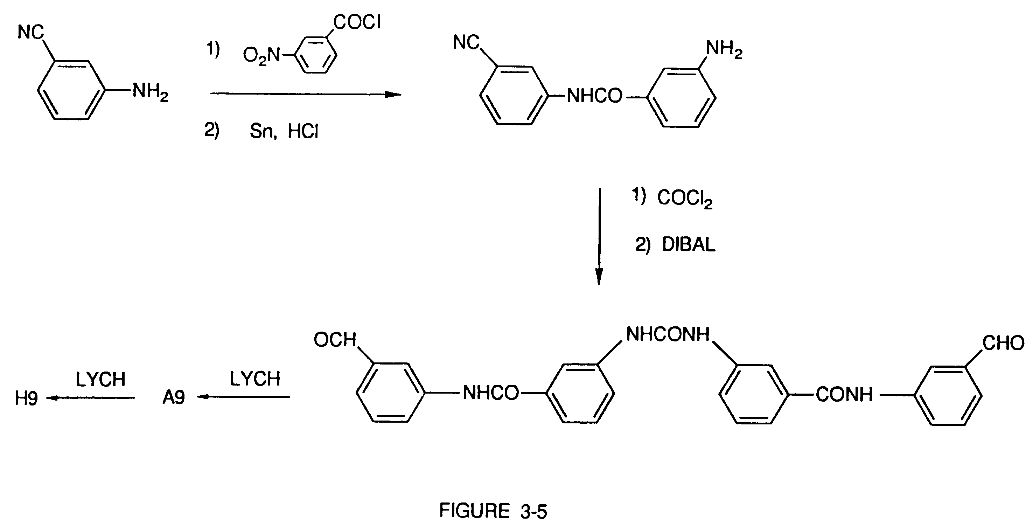

When conjugated with LY-CH, the conjugate has the formula designated H2: 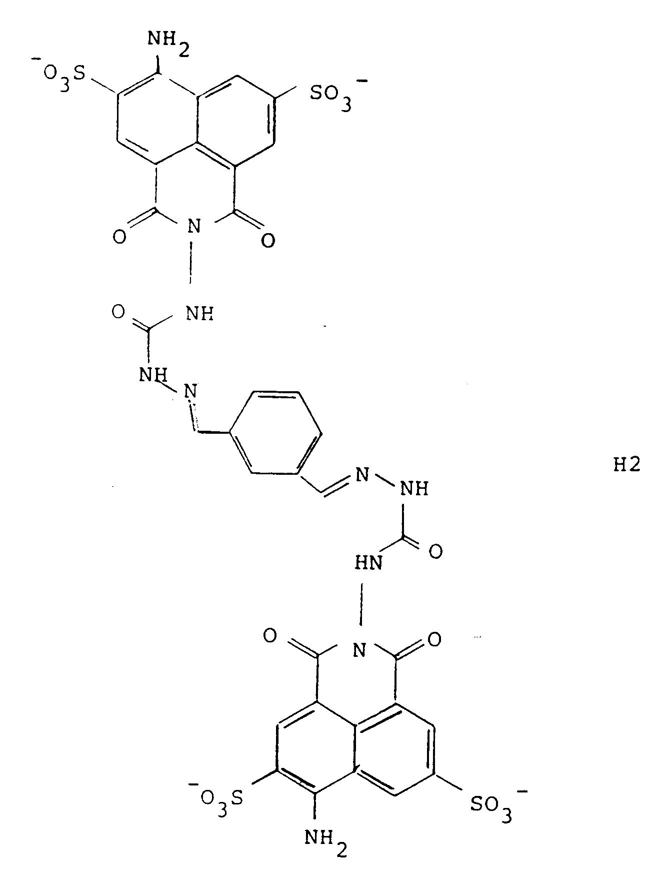
Thus, a series of preferred precursors and conjugates are constructed wherein the compound of formula (3) is a pharmaceutically acceptable salt of Lucifer Yellow and the compound of formula (2) is derived from Lucifer Yellow and Y of the various formulas shown in Figure 2. These compounds of formula (2) are designated A1-A9, wherein these precursors result in conjugates with LY-CH to give the corresponding dihydrazones (semicarbazones) of formulas H1 through H9. The synthesis of all of these embodiments is outlined in Figure 3. As shown in Figure 3, the dialdehyde of the embodiments of Y shown as formulas 1-9 in Figure 2 is first prepared and reacted with 1 molar equivalent of Lucifer Yellow to obtain the precursors A1-A9; subsequent reaction with the other precursor, LY-CH results in the toxic and antiviral conjugates H1-H9. The reaction with the second mole of LY-CH, of course, can, in the preferred case, take place in situ.

With respect to the simple hydrazones (semicarbazones) of Formula 4, preferred forms of the conjugates also include those wherein R represents the tricyclic residue of Lucifer Yellow--i.e., Z is NH₂ at the position shown.

Preferred embodiments of R¹ are H and lower alkyl (1-4), most preferably H. By alkyl is meant a saturated hydrocarbyl of the indicated number of carbon atoms which is in a straight or branch chain form, such as methyl, ethyl, n-propyl, i-propyl, t-butyl, n-butyl, i-hexyl, n-hexyl, and 3-methylpentyl.

With respect to R², although R² can be alkyl, expecially if R¹ is not H, conjugates in which R² is aryl are preferred. By aryl is meant a 6-10 membered monocyclic or fused bicyclic ring system which has aromatic character. The ring may contain only carbons as ring members, or may contain one or more heteroatoms such as N. The aryl substituents are, thus, typically, moieties such as phenyl, pyridyl, napthyl, quinolyl, pyrimidyl, and the like. The aryl substituents may contain 1-2 nonhydrogen substitutions which do not interfere with the pharmacological effect of the conjugate. Typical substituents include, but are not limited to, lower alkyl, lower alkoxy, hydroxy, amino, thio, alkyl amino, alkyl thio, halo, carboxy, carbalkoxy, or CHO.

The substituent R² may also include multiple "linked" aryl (or, perhaps, more properly, arylene) substituents wherein a total of 1-5 aryl moieties of 6-10C are linked through nonaromatic substituents or through a covalent bond to form an extended chain of aryl groups. Perhaps the simplest example of this is the embodiment of R² which is biphenyl wherein two phenyl groups are linked through a single covalent bond. Other examples of this type of "necklace" of aryl moieties include instances wherein phenyl groups are linked through -CONH- or -NHCONH-. Other types of linkage include hydrocarbylene linkages and conjugation-type linkages through -CH=CH-. As is the case for a single aryl substituent, the aryl groups forming the extended chain may be substituted with 1-2 noninterfering substituents. A particularly preferred substituent is -CHO. Particularly preferred also are those embodiments wherein at least two linked aryl groups are present in the substituent R².

Thus, particularly preferred as conjugates are those derived from Lucifer Yellow CH itself, including those in the form of any pharmaceutically acceptable salt, and the compound of the formula: 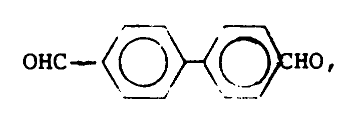
i.e., those of the formula 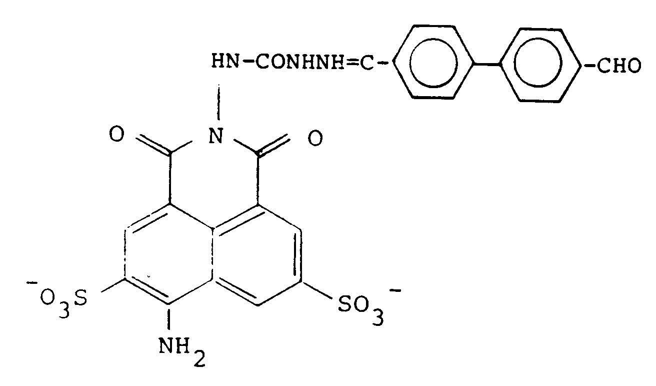
Compound A4 may be synthesised from commercially available reagents. The commercially available compound 4,4'-dicyanobiphenyl is reduced in DIBAL and hydrolyzed to obtain the dialdehyde. The dialdehyde is then reacted with the lithium salt of Lucifer Yellow in a controlled reaction to obtain the single compound A4, which is then isolated using standard chromatographic methods. The lithium salt, is, if desired, converted to alternate pharmaceutically acceptable nontoxic salts.

### B. Selectivity for Monocytes and Derivatives

Lucifer Yellow itself is a nontoxic, fluorescent, membrane-impermeable dye (Stewart, W.W., Cell (1978) 14:741) which is taken up by monocyte/macrophage endosomes through pinocytosis and is concentrated in the lysosomes. The rate of pinocytosis and accumulation in lysosomes is higher for macrophages, especially activated macrophages, than for other cell types such as fibroblasts (Besterman, J.M., et al, J Cell Biol (1981) 91:916-917; Steinman, R.M., et al, J Cell Biol (1976) 68:665-687) and does not bind to proteins to a substantial degree. The analogs of Lucifer Yellow which are within the scope of this invention exhibit similar properties. Because no specific receptor interaction is involved in the uptake of Lucifer Yellow by monocytes, these analogs which retain the membrane impermeable nature of LY-CH are similarly taken up by the lysosomes. In addition, unlike suramin, the LY-CH and analog-derived precursors of the invention are not bound to serum proteins. Thus, their bioavailability is not diminished in this way.

Thus, the selectivity of precursors and conjugates derived from Lucifer Yellow and its analogs are expected to home to the virus-tropic cells, such as monocytes, rather than to the non-tropic cells, for example neutrophils. In addition, as the Lucifer Yellow-derived materials are concentrated in the lysosomes, where the pH is relatively low (4-5) reaction to form the hydrazone or semicarbazone is favored.

As described in PCT application WO88/00047, concentration of the precursors in the desired target cells results in a magnified selectivity when the toxic material is formed by a second order reaction from the precursors. Thus, for example, a three-fold higher concentration of each precursor in the target cells as compared to other destinations would result in a nine-fold concentration differential between the target and the remaining tissues. In the present application, this is enhanced by the favorable conditions found in the targets for formation of the conjugate-i.e., the conditions are such that the rate constant for the forward reaction is increased.

### C. Formulation and Administration

As the components are to be administered as pharmaceuticals, they are formulated in a conventional manner and administered either localized at the targeted site or sytemically. As is generally the case for self-assembling, pharmaceutically active products, administration at the intended site of activity enhances the assembly of the biologically active conjugate by the high local concentrations of the components, and diffusion of the components away from the target site automatically diminishes their tendency to combine. Generally known methods of formulation are employed, including sustained-release matrices, conventional excipients, and simple solutions, as described, for example, in Remington's Pharmaceutical Sciences (latest edition), Mack Publishing Company, Easton, Pennsylvania. In some instances, the conjugate formed by the precursors of the invention can be administered in a form in which conjugation has already taken place. Thus, the resulting pharmaceutically active conjugate can itself be formulated and administered to the patient. However, it is preferable to administer the precursors separately, either simultaneously and in the same location, or in different locations at the same time, or in the same location at different times.

Systemic administration is feasible in using the precursors as treatment for HIV infection, since it is known that LY-CH is taken up and concentrated in the endosomes and lysosomes of macrophages. This has been reported for murine thiol-elicited macrophages. These organelles have lower pH (about 5), thus enhancing the production of the conjugated semicarbazone. This results in a concentration of the conjugate in T-cells which contact macrophages, as opposed to kidney or other parts of the body. The compound thus tends to accumulate and react in locations where the virus infection is likely to be found.

The dosage level required depends, of course, on the choice of specific end-products and/or components. For most embodiments, a dosage of the conjugate on the order of 0.1-5 g/week appears suitable; for LY-CH and the complementary preferred compounds set forth above, i.e., the precursors administration of 100mg-3g/week of each component is appropriate.

The preformed conjugates can be administered intravenously, intramuscularly, orally, or intraperitoneally, at about 1-1000 mg/kg body weight.

The dosage level required depends, of course, on the choice of specific conjugate, as well as the nature of the subject being treated for retroviral infection and the magnitude of the infection or the potential therefor. Administration may have either therapeutic or prophylactic effects or both. For most embodiments, a dosage of the conjugate on the order of 0.1-5 g/week appears suitable.

In a preferred approach, by derivatizing the precursors so as to administer them in particulate form, for example, by conjugating them to a carbohydrate, selective killing of the macrophage cells which harbor the virus can be accomplished. Because the drugs are perceived as particles, they will be readily attacked by the macrophages.

The following examples are intended to illustrate, but not to limit, the invention.

### Example 1

### Effect of Precursors and Conjugates on HIV

The test culture system uses MT-2 cells, which are a T-cell-derived leukemic cell line derived from a person infected with HTLV-1 (Richman, D.D., et al, J Exp Med (1987) 166:1144-1149). When these cells are cultured and then infected with HIV-1, they form multinucleated macrocells called syncitia by self-fusion. These syncitia can be observed microscopically and quantitated.

Infected cell stock is prepared by adding HIV to 10 ml MT-2 cell suspension containing 6 x 10⁵ cells/ml in RPMI-1640 medium supplemented with 2 mM glutamine, polybrene, 100 U/ml penicillin, 100 U/ml streptomycin, and 10% fetal calf serum. To 10 ml of MT-2 suspension is added 60 ul of HIV virus concentrate (10⁷ "tissue 50% infectious disease units" (TCID₅₀)/ml, strain 5735).

Solutions of the precursor or conjugate to be tested at 2x their final concentration were diluted with the infected cell stock in 24 well tissue culture plates. 100 ul each of the drug stock and infected cell stock were used. After 3-4 days incubation at 37°C, 5% CO₂, 100% humidity, the cells were checked for syncitia formation and rated on a scale from zero (no syncitia) to four (a maximum viral cytopathic effect--i.e., no antiviral activity).

Lucifer Yellow-CH (LY-CH) concentrations of 10 µM, 40 µM and 200 uM were tested; all showed no antiviral effect and received a rating of 4; LY-CH was not antiviral even at 800 uM. A2 was tested at 10 µM, 40 µM and 200 µM; there was no antiviral effect at the two lower concentrations, but a slight effect (a rating of 3) at 200 µM; at 400 µM and 800 µM A2 completely inhibited syncitia formation. When the drug contained a combination of 100 µM LY plus 100 µM A2, a marked antiviral effect (0 and 1 in two separate determinations) was found. Lower concentration levels of the combined precursors (20 µM and 5 µM of each) gave no antiviral effect.

In the foregoing results, stock solutions used to prepare the combination drugs at concentrations of 10-fold the final concentration of the drug solution added to the wells were incubated together at 25°C for an hour before dilution to begin the incubation with the cell cultures.

In a subsequent experiment, conducted similarly, this recombination was not done, so no conjugation prior to addition to the culture could occur. Under these conditions, higher concentrations of the combinations were required. Concentrations of 100 µM A2 plus 100 µM LY-CH in this assay, without precombination, did not show an antiviral effect. However, when the concentration of both components was raised to 200 µM, a moderate (2-3) antiviral effect was exhibited, and combinations where both concentrations were 400 µM were strongly antiviral (0). However, 400 µM A2 alone and 800 µM A2 also show pronounced antiviral effects, and 200 µM A2 alone, a moderate one (3). At all concentrations of LY-CH tested (100-800 µM) no antiviral effect was found.

Similar assays, but using HIV viral antigen expression in MDM as criterion for antiviral activity, were conducted. In these assays, the expression was rated from 0 (complete inhibition) to 180 (no drug effect). In this assay, the following results were obtained, as shown in Table 1:

**Table 1**

| | |
|---|---|
| 40 µM LY | 170 |
| 200 µM LY | 95 |
| 40 µM A2 | 200 |
| 200 µM A2 | 135 |
| 100 µM LY + 100 µM A2 | 83 |

As shown in the table, according to this assay, the effect of LY can be dramatically enhanced by the addition of A2.

None of the compounds used was cytotoxic at the concentrations used hereinabove to either cat kidney epithelial cells after 4 days or to human monocyte-derived macrophages after 5 days. However, preincubated 150 µM A2+250 µM LY-CH resulted in 50% inhibition of all growth over periods where unreacted components showed no effect.

### Example 2

### Effect of Compound A4 on HIV and FIV

Compound A4 was first tested for its ability to inhibit reverse transcriptase isolated either from HIV-1 or from FIV using a standard protocol. In each case, compound A4 was more potent than suramin in inhibiting the enzyme. The approximate IC₅₀ values are 200 µM against HIV-1 and 20 µM against FIV.

Compound A4 was also tested for its ability to inhibit the growth of FIV in a culture containing feline kidney cells using standard assays. Using a concentration of 30 µg/ml compound A4, FIV growth and proliferation were virtually completely inhibited within 20 days following infection; administration of 20 µg/ml compound A4 resulted in corresponding inhibition 40 days thereafter. Smaller concentrations had similar effects but were less dramatic.

The effect of compound A4 (20 µg/ml) on the viability of infected cells was also investigated. This concentration of compound A4 was able to reestablish 100% viability of the infected cells within 50 days of infection.

Finally, it has been shown that the macrophage cell line U937 takes up and retains compound A4.

In an in vivo test, compound A4 was injected into two mice intraperitoneally at a dose of 60 mg/kg. No apparent toxicity was evident 15 days later, showing that the compound is relatively non-toxic.

## Claims

1. A compound of formula (1)
R-NHCONHN=CH-Y-CH=NNHCONHR (1)
wherein R has the formula: 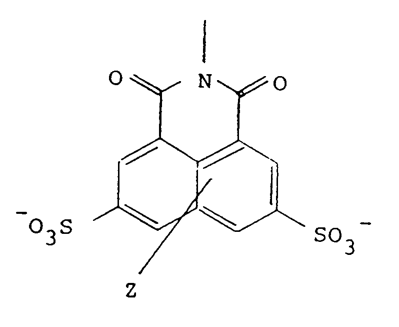 in which Z represents one or two non-interfering substituents, and
Y is selected from the group consisting of the moieties 2-1 to 2-9:

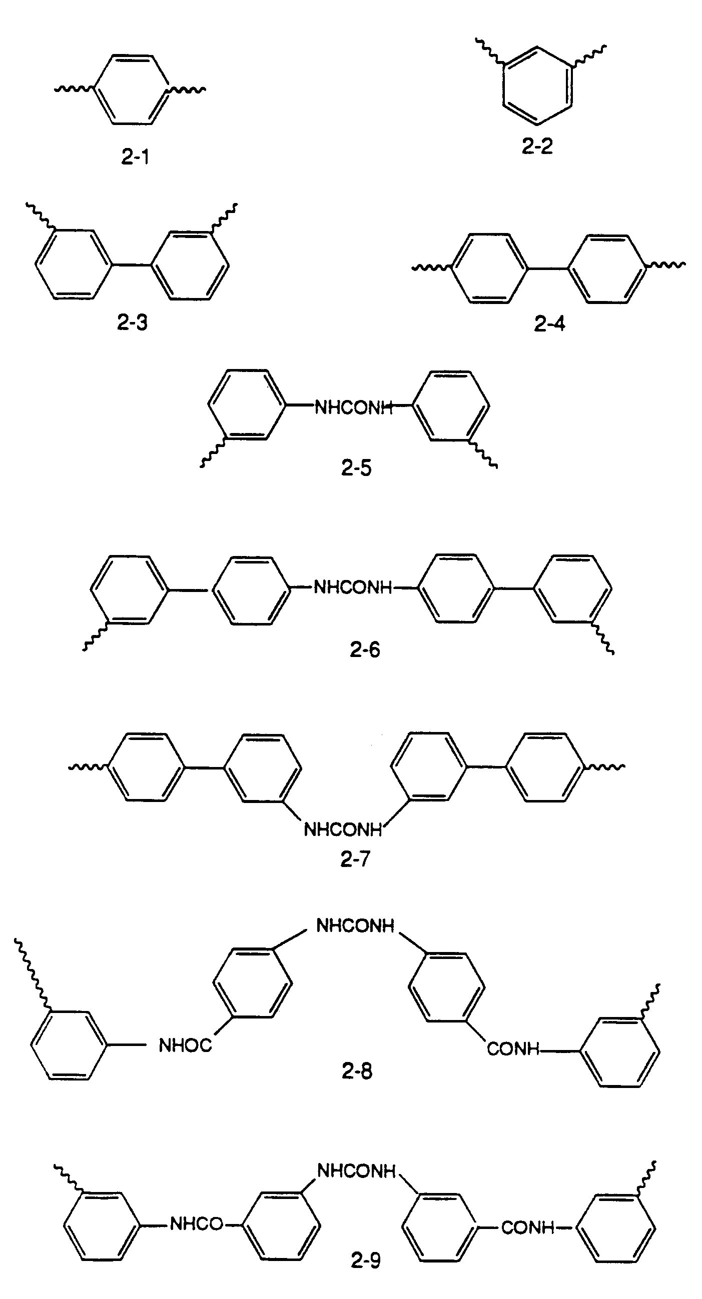

2. A compound according to claim 1 in which Z is one or two non-interfering substituents which are lower alkyl, lower alkoxy, hydroxy, amino, thio, alkylamino, alkylthio, or halo or a pharmaceutically acceptable salt thereof.

3. A compound according to claim 2 wherein R is 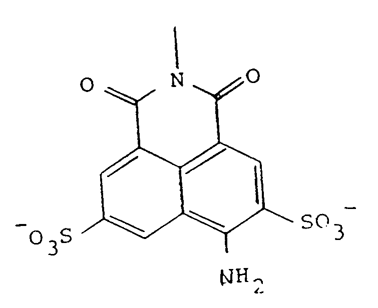 as the pharmaceutically acceptable salt.

4. A compound according to claim 1, 2 or 3 in which Y is

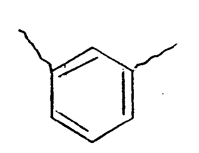

5. A compound according to claim 4, designated H2, which is of the formula:

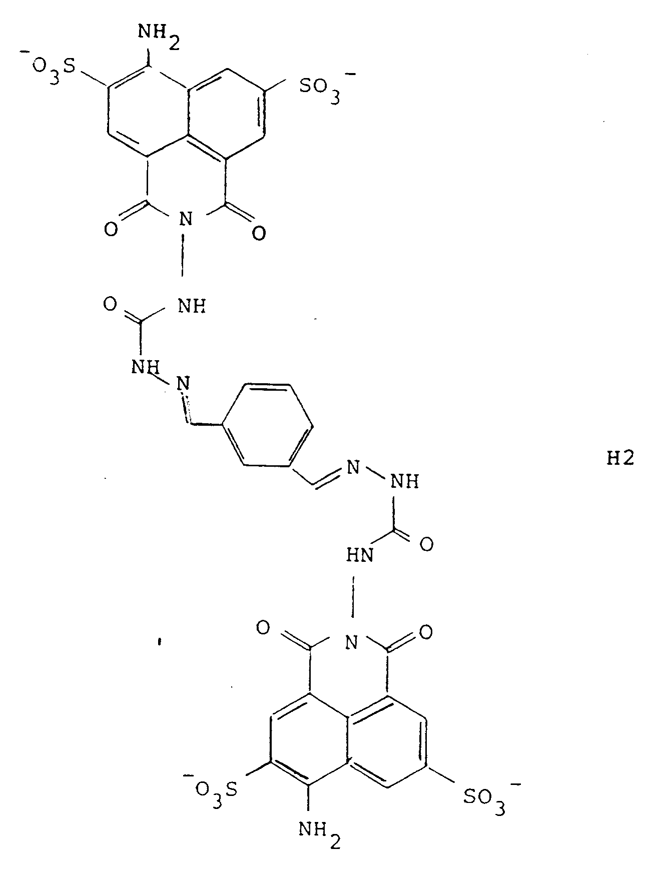

6. A process for preparing a compound of formula (1) as defined in claim 1 which comprises contacting a compound of formula (2) with a compound of formula (3):
R-NHCONHN=CH-Y-CHO (2)
R-NHCONHNH₂ (3)
in which R and Y are as defined in any one of the preceding claims.

7. A pharmaceutical composition useful in the treatment of HIV infection which contains, as active ingredients, precursors of a compound of formula (1) as defined in any of claims 1 to 5, which precursors are capable of self-assembly into the compound of formula (1) in the presence of virus-infected cells.

8. A pharmaceutical composition useful in the treatment of retroviral infection which contains, as active ingredients, compounds of the formula:
R-NHCONHN=CH-Y-CHO (2)
and
R-NHCONHNH₂ (3)
wherein R and Y are as defined in any of claims 1 to 5.

9. A composition according to claim 8 wherein the compounds of formulae (2) and (3), are designated A2 and LY-CH respectively, and are of formulae 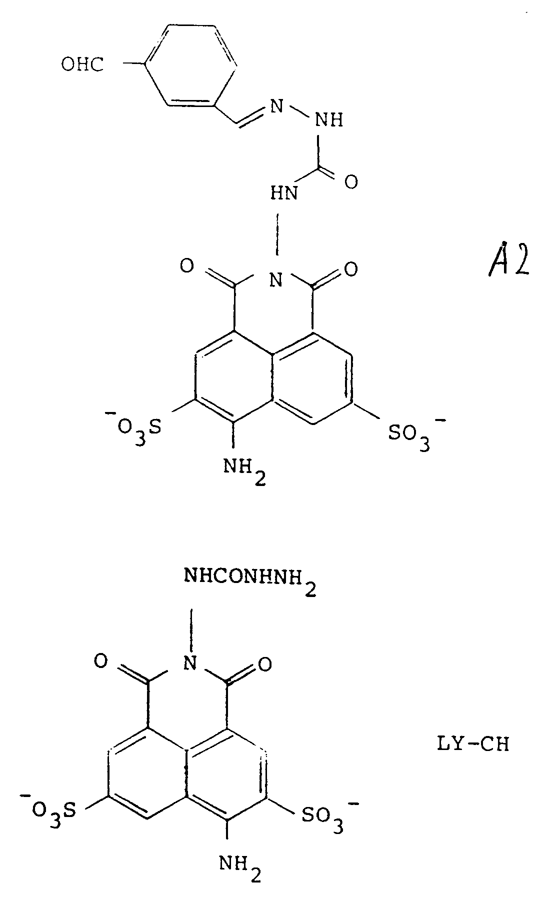 and the compound of formula (1) is H2, as defined in claim 5.

10. A combination of precursors of a compound of formula (1) as claimed in any of claims 1 to 5, which are capable of self assembly into the compound of formula (1) in the presence of virus-infected or virus-susceptible cells for use in the treatment of retroviral infection in a human subject.

11. A combination of precursors according to claim 10 of the formulae (2) and (3) as defined in claim 8 or 9.

12. Use of a precursor of a compound of formula (1) as claimed in any one of claims 1 to 5 in combination with a further precursor of a compound of formula (1), which precursors are capable of self-assembly into the compound of formula (1) in the present of virus-infected or virus susceptible cells, in the manufacture of a medicament for use in the treatment of retroviral infection in a human subject.

13. Use according to claim 12 of a precursor which is a compound of formula (3)
R-NHCONHNH₂ (3)
in combination with a further precursor which is a compound of formula (2)
R-NHCONHN=CH-Y-CHO (2)
in which R and Y are as defined in any one of claims 1 to 5.

14. Use according to claim 13 of LY-CH in combination with A2 where LY-CH and A2 are as defined in claim 9.

15. A compound for use in the treatment of retroviral infection in a human subject, which is a compound of formula (2)
R-NHCONHN=CH-Y-CHO (2)
for use in combination with a further precursor which is a compound of formula (3)
R-NHCONHNH₂ (3)
in which R and Y are as defined in any one of claims 1 to 5.

16. A compound according to claim 15 which is A2, for use in combination with the precursor LY-CH where A2 and LY-CH are as defined in claim 9.

17. A compound of formula (4) 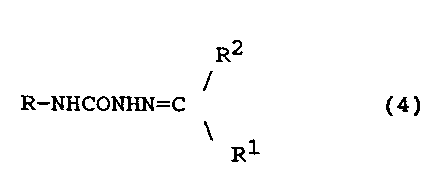 wherein R has the formula: 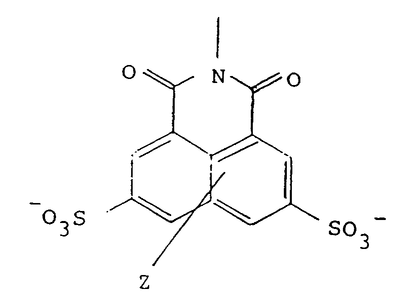 in which Z represents one or two non-interfering substituents;
and wherein R¹ is H or alkyl (1-6C); and
R² is alkyl (1-6C) or aryl (6-10C) or linked aryl (6-10C) containing 2-5 such aryl groups, said alkyl or aryl groups being unsubstituted or substituted by 1 or 2 non-interfering substituents,
or a pharmaceutically acceptable salt thereof.

18. A compound according to claim 17 in which Z is one or two non-interfering substituents which are lower alkyl, lower alkoxy, hydroxy, amino, thio, alkylamino, alkylthio, or halo; or a pharmaceutically acceptable salt thereof.

19. A compound according to claim 17 or 18 in which R² is alkyl (1-6C) or aryl (6-10C) or linked aryl (6-10C) containing 2-5 such aryl groups, said aryl groups being unsubstituted or substituted by 1 or 2 non-interfering substituents which are lower alkyl, lower alkoxy, hydroxy, amino, thio, alkylamino, alkylthio, halo, carboxy, carbalkoxy or -CHO; or a pharmaceutically acceptable salt thereof.

20. A compound according to claim 19 of the formula: 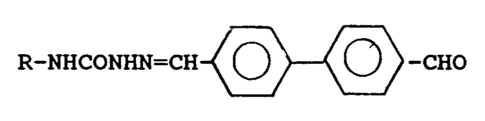 or a pharmaceutically acceptable salt thereof.

21. A compound according to claim 20 of the formula 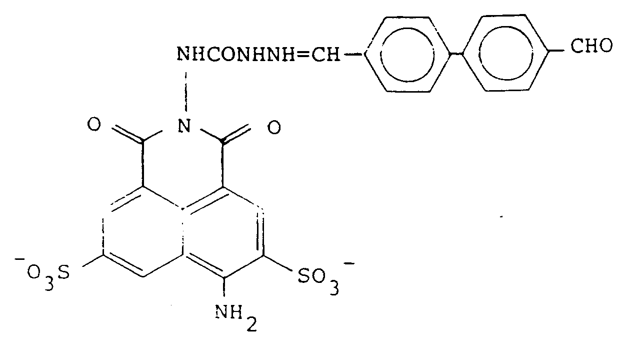 or a pharmaceutically acceptable salt thereof.

22. A pharmaceutical composition useful in the prevention or treatment of retroviral infection which contains, as active ingredient, an effective amount of a compound according to any one of claims 17 to 21.

23. A method to synthesize a compound according to any one of claims 17 to 21, which method comprises contacting a compound the formula (3)
R-NHCONHNH₂ (3)
wherein R is as defined in any of claims 17 to 21, with a compound of the formula (5) 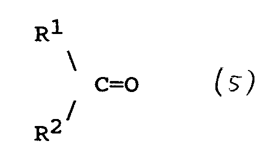 wherein R¹ and R² are as defined in any of claims 17 to 21; and recovering the compound of formula (4) as defined in claim 17.

24. A compound according to any one of claims 17 to 21 for use in treatment or prevention of retroviral infection in an animal subject.

## Patentansprüche

1. Verbindung der Formel (1)
R-NHCONHN=CH-Y-CH=NNHCONHR (1),
worin R die Struktur 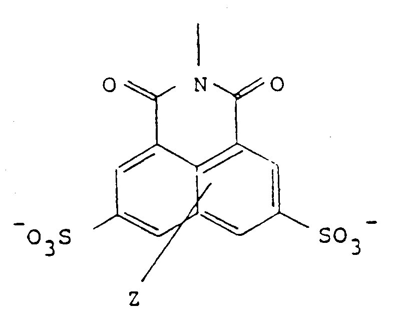 aufweist, in welcher Z einen oder zwei keine Reaktion auslõsende Substituenten verkõrpert und
Y ausgewãhlt ist aus der Gruppe, bestehend aus den Komponenten 2-1 bis 2-9:

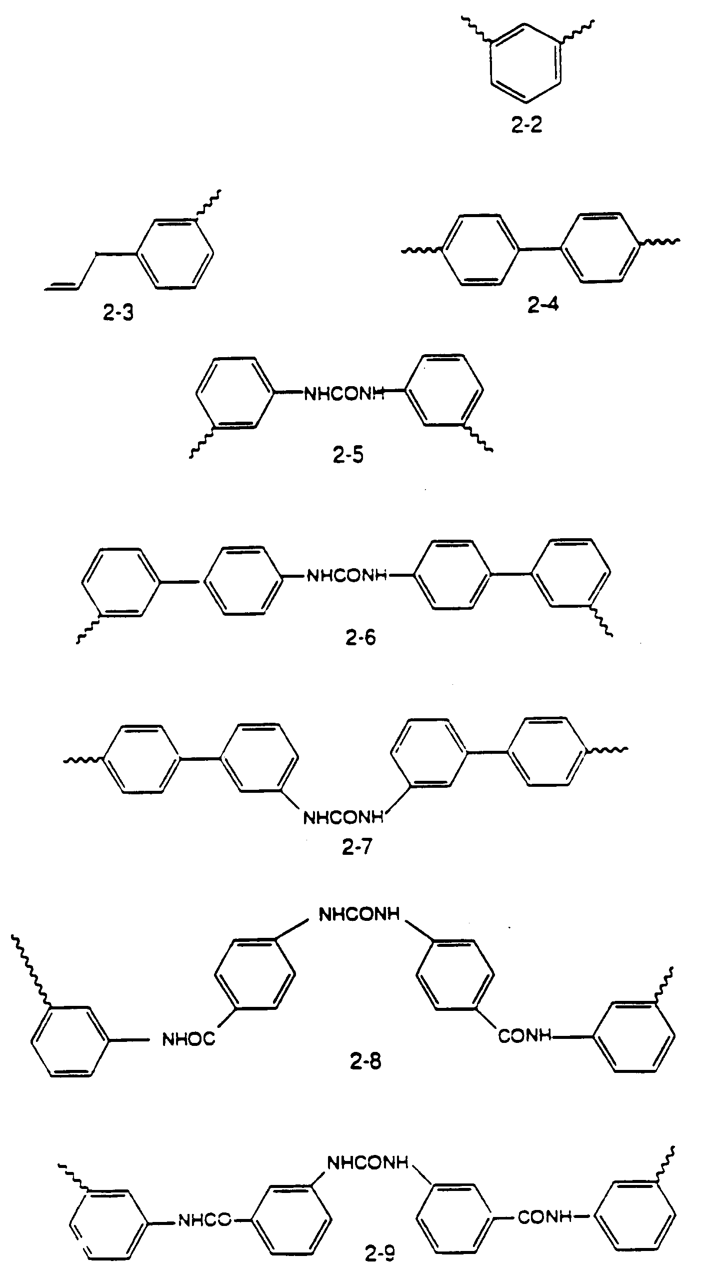

2. Verbindung nach Anspruch 1, in welcher Z einen oder zwei keine Reaktion auslõsende Substituenten verkõrpert, die kurzkettiges Alkyl, kurzkettiges Alkoxy, Hydroxy, Amino, Thio, Alkylamino, Alkylthio oder Halogen, oder ein pharmazeutisch geeignetes Salz davon sind.

3. Verbindung nach Anspruch 2, worin R als das pharmazeutisch geeignete Salz 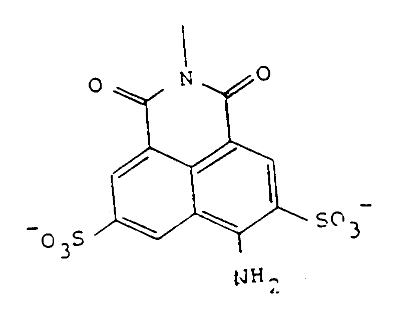 ist.

4. Verbindung nach Anspruch 1, 2 oder 3, in welcher Y 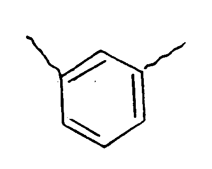 ist.

5. Verbindung nach Anspruch 4, bezeichnet als H2, welche die folgende Struktur aufweist:

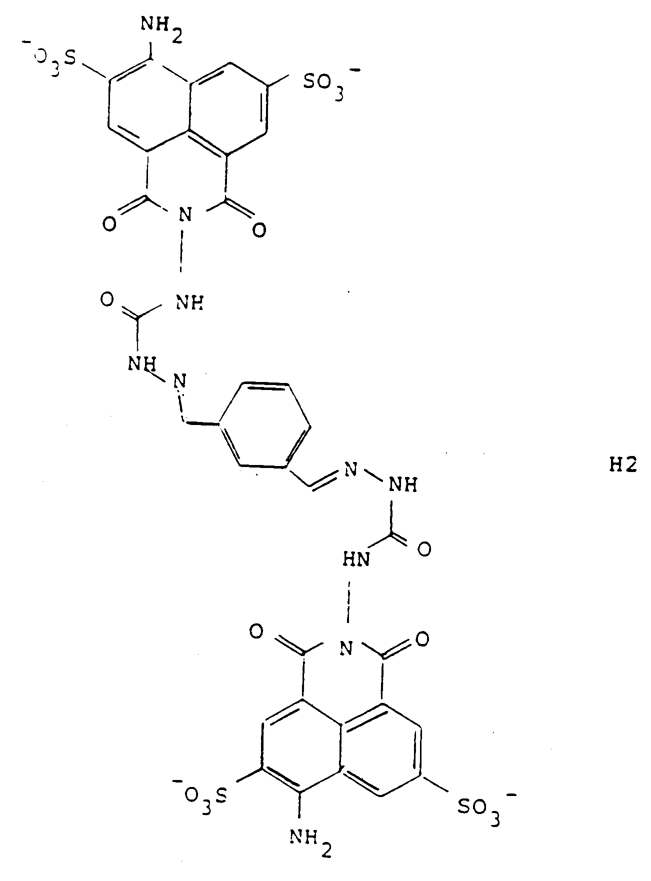

6. Verfahren zur Herstellung einer Verbindung der Formel (1), wie sie in Anspruch 1 definiert ist, welches das Inkontaktbringen einer Verbindung der Formel (2) mit einer Verbindung der Formel (3) umfaßt:
R-NHCONHN=CH-Y-CHO (2)
R-NHCONHNH₂ (3),
in welchen R und Y wie in einem der vorangegangenen Ansprüche definiert sind.

7. Pharmazeutische Zusammensetzung, die bei der Behandlung einer HIV-Infektion nützlich ist, welche Vorlãufer einer Verbindung der Formel (1), wie sie in einem der Ansprüche 1 bis 5 definiert ist, als Wirkstoffe enthãlt, wobei diese Vorlãufer zur selbstãndigen Anordnung zu der Verbindung der Formel (1) in Gegenwart Virus-infizierter Zellen fãhig sind.

8. Pharmazeutische Zusammensetzung, die bei der Behandlung einer Retrovirus-Infektion nützlich ist, welche Verbindungen der Formeln
R-NHCONHN=CH-Y-CHO (2)
und
R-NHCONHNH₂ (3)
als Wirkstoffe enthãlt, worin R und Y wie in einem der Ansprüche 1 bis 5 definiert sind.

9. Zusammensetzung nach Anspruch 8, worin die Verbindungen der Formeln (2) und (3) als A2 bzw. LY-CH bezeichnet sind und die Strukturen 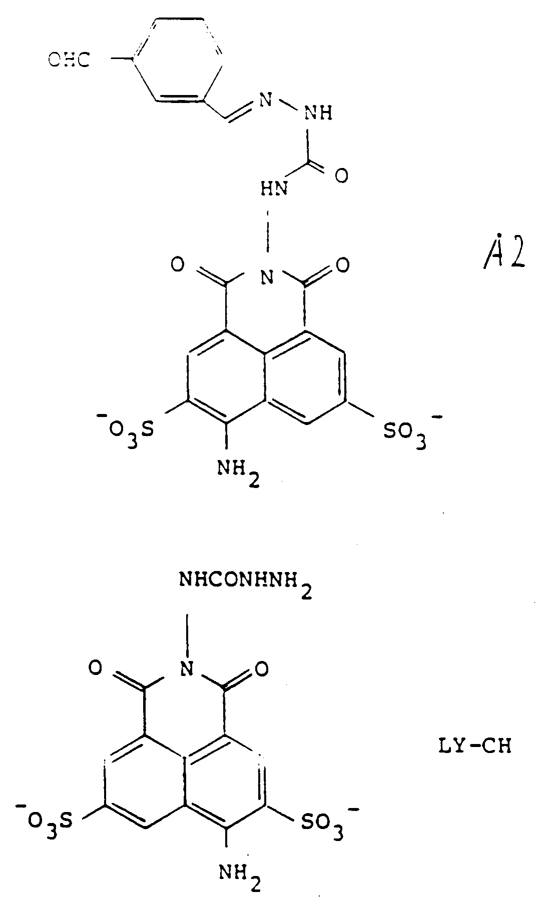 aufweisen und die Verbindung der Formel (1) H2 ist, wie in Anspruch 5 definiert.

10. Kombination von Vorlãufern einer Verbindung der Formel (1), wie sie in den Ansprüchen 1 bis 5 beansprucht ist, die zur selbstãndigen Anordnung zu der Verbindung der Formel (1) in Gegenwart Virus-infizierter oder Virus-anfãlliger Zellen fãhig sind, zur Verwendung bei der Behandlung einer Retrovirus-Infektion beim Menschen.

11. Kombination von Vorlãufern nach Anspruch 10 der Formeln (2) und (3), wie sie in Anspruch 8 oder 9 definiert sind.

12. Verwendung einer Vorlãufers einer Verbindung der Formel (1), wie sie in den Ansprüchen 1 bis 5 beansprucht ist, in Kombination mit einem weiteren Vorlãufer einer Verbindung der Formel (1), welche Vorlãufer zur selbstãndigen Anordnung zu der Verbindung der Formel (1) in Gegenwart Virus-infizierter oder Virus-anfãlliger Zellen fãhig sind, bei der Herstellung eines Medikaments, das bei der Behandlung einer Retrovirus-Infektion beim Menschen Verwendung findet.

13. Verwendung nach Anspruch 12 eines Vorlãufers, welcher eine Verbindung der Formel (3)
R-NHCONHNH₂ (3)
ist in Kombination mit einem weiteren Vorlãufer, welcher eine Verbindung der Formel (2)
R-NHCONHN=CH-Y-CHO (2)
ist, in welchen R und Y wie in Ansprüchen 1 bis 5 definiert sind.

14. Verwendung nach Anspruch 13 von LY-CH in Kombination mit A2, wobei LY-CH und A2 wie in Anspruch 9 definiert sind.

15. Verbindung zur Verwendung bei der Behandlung einer Retrovirus-Infektion beim Menschen, welche eine Verbindung der Formel (2)
R-NHCONHN=CH-Y-CHO (2)
ist, zur Verwendung in Kombination mit einem weiteren Vorlãufer, welcher eine Verbindung der Formel (3)
R-NHCONHNH₂ (3)
ist, in welchen R und Y wie in Ansprüchen 1 bis 5 definiert sind.

16. Verbindung nach Anspruch 15, welche A2 ist, zur Verwendung in Kombination mit dem Vorlãufer LY-CH, wobei A2 und LY-CH wie in Anspruch 9 definiert sind.

17. Verbindung der Formel (4) 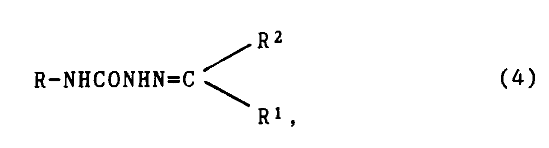 worin R die Struktur 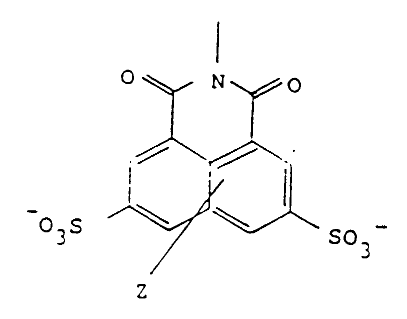 aufweist, in welcher Z einen oder zwei keine Reaktion auslõsende Substituenten verkõrpert;
und worin R¹ H oder Alkyl (1-6C) ist; und R² Alkyl (1-6C) oder Aryl (6-10C) oder gebundenes Aryl (6-10C) ist, das 2-5 solcher Arylgruppen enthãlt, wobei die Alkyl- oder Arylgruppen unsubstituiert oder durch 1 oder 2 keine Reaktion auslõsende Substituenten substituiert sind;
oder ein pharmazeutisch geeignetes Salz davon.

18. Verbindung nach Anspruch 17, in welcher Z ein oder zwei keine Reaktion auslõsende Substituenten sind, die kurzkettiges Alkyl, kurzkettiges Alkoxy, Hydroxy, Amino, Thio, Alkylamino, Alkylthio oder Halogen sind; oder ein pharmazeutisch geeignetes Salz davon.

19. Verbindung nach Anspruch 17 oder 18, in welcher R² Alkyl (1-6C) oder Aryl (6-10C) oder gebundenes Aryl (6-10C) ist, das 2-5 solcher Aryl-Gruppen enthält, wobei die Arylgruppen unsubstituiert oder durch 1 bis 2 keine Reaktion auslõsende Substituenten substituiert sind, welche kurzkettiges Alkyl, kurzkettiges Alkoxy, Hydroxy, Amino, Thio, Alkylamino, Alkylthio, Halogen, Carboxy, Carbalkoxy oder -CHO sind; oder ein pharmazeutisch geeignetes Salz davon.

20. Verbindung nach Anspruch 19 der Formel 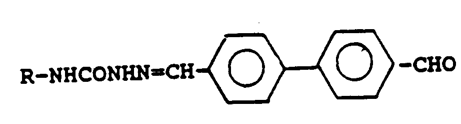 oder ein pharmazeutisch geeignetes Salz davon.

21. Verbindung nach Anspruch 20 der Formel 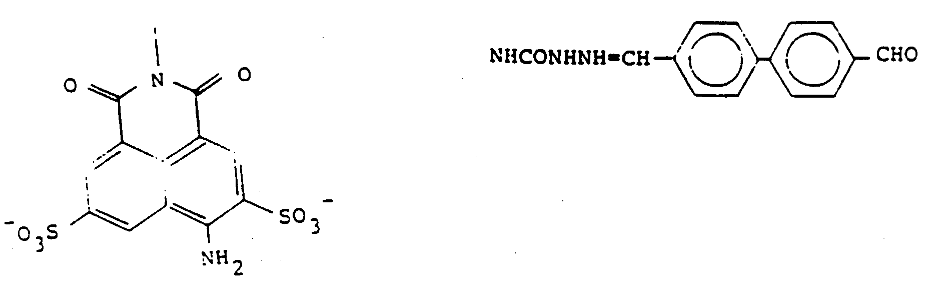 oder ein pharmazeutisch geeignetes Salz davon.

22. Pharmazeutische Zusammensetzung, die zur Vorbeugung gegen oder Behandlung einer Retrovirus-Infektion nützlich ist, welche eine Wirkmenge einer Verbindung nach einem der Ansprüche 17 bis 21 als Wirkstoff enthãlt.

23. Verfahren zur Synthetisierung einer Verbindung nach einem der Ansprüche 17 bis 21, welches das Inkontaktbringen einer Verbindung der Formel (3)
R-NHCONHNH₂ (3)
umfaßt, worin R wie in einem der Ansprüche 17 bis 21 definiert ist, mit einer Verbindung der Formel (5) 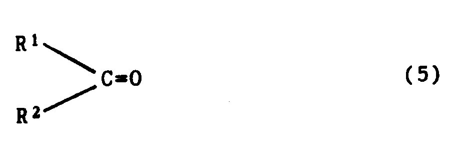 worin R¹ und R² wie in einem der Ansprüche 17 bis 21 definiert sind,
und das Wiedergewinnen der Verbindung der Formel (4), wie sie in Anspruch 17 definiert ist.

24. Verbindung nach einem der Ansprüche 17 bis 21, die bei der Behandlung oder Vorbeugung gegen eine Retrovirus-Infektion beim Tier Verwendung findet.

## Revendications

1. Composé de formule (1)
R-NHCONHN=CH-Y-CH=NNHCONHR (1)
dans laquelle R répond à la formule : 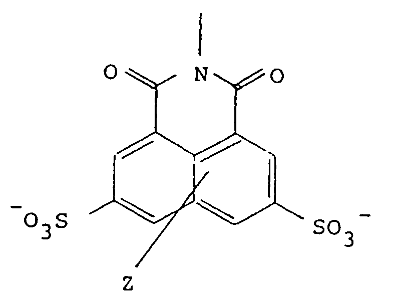 dans laquelle Z représente un ou deux substituants non interférants, et
Y est choisi dans le groupe constitué par les entités 2-1 à 2-9 :

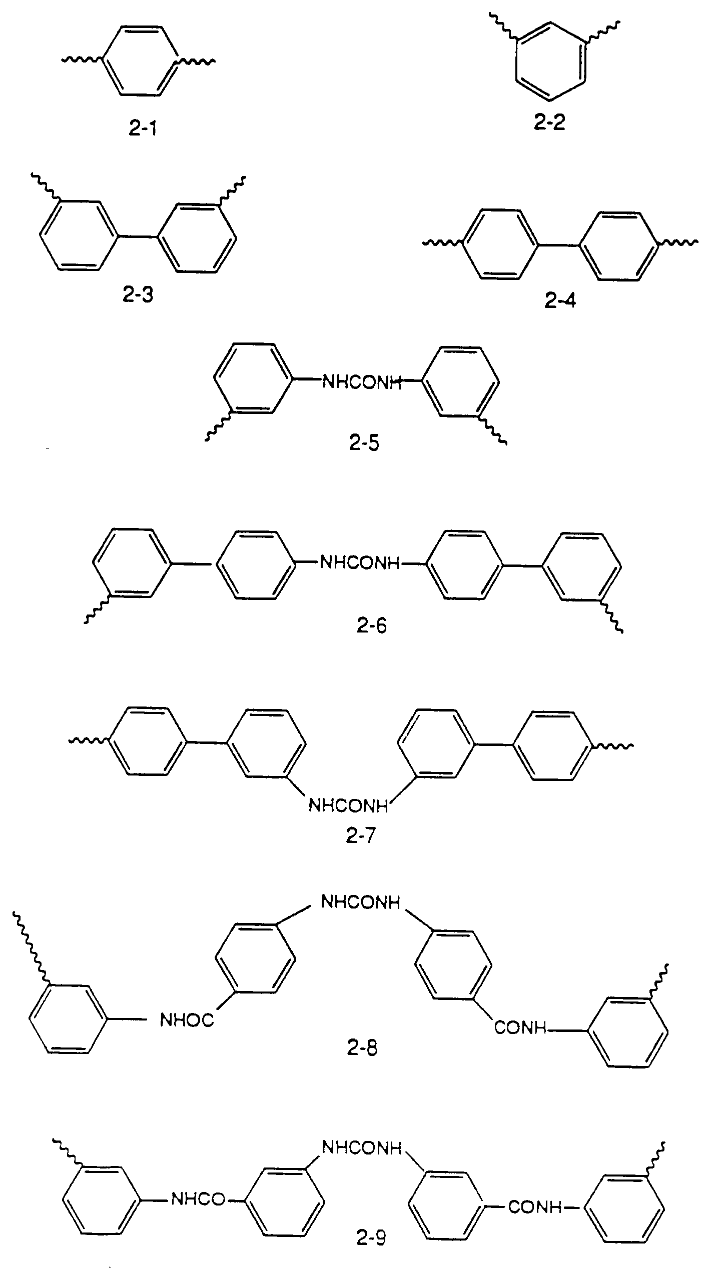

2. Composé selon la revendication 1, dans lequel Z représente un ou deux substituants non interférants, qui sont des groupes alkyle inférieur, alcoxy inférieur, hydroxy, amino, thio, alkylamino, alkylthio ou halogéno, ou un de leurs sels pharmaceutiquement acceptables.

3. Composé selon la revendication 2, dans lequel R représente 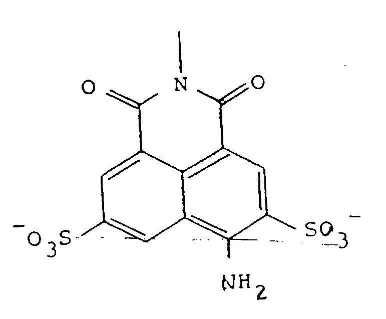 comme sel pharmaceutiquement acceptable.

4. Composé selon la revendication 1, 2 ou 3, dans lequel Y représente

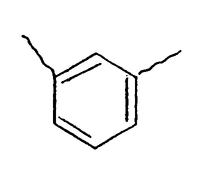

5. Composé selon la revendication 4, désigné par H2, qui répond à la formule :

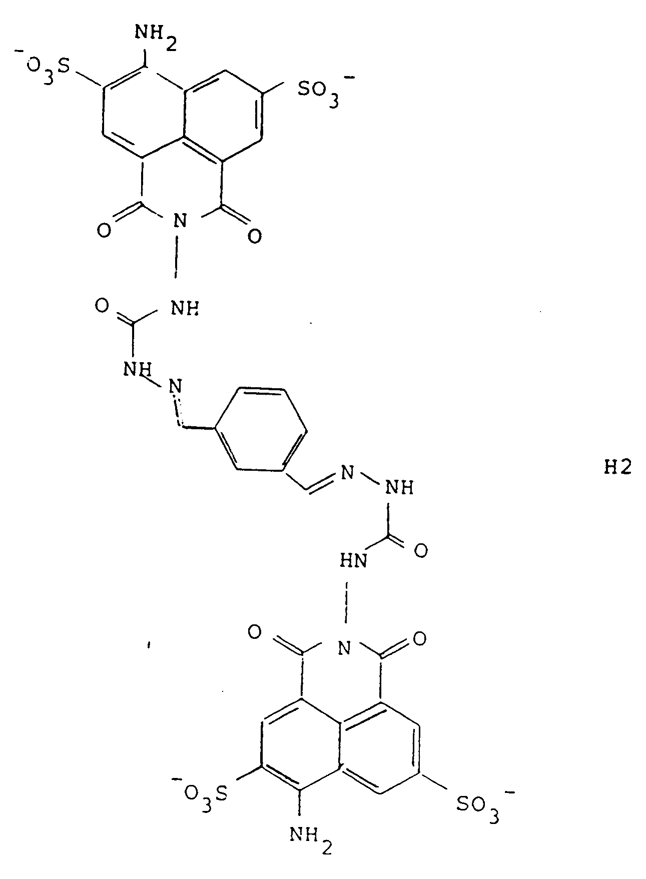

6. Procédé de préparation d'un composé de formule (1), tel que défini dans la revendication 1, qui comprend les étapes consistant à mettre en contact un composé de formule (2) avec un composé de formule (3) :
R-NHCONHN=CH-Y-CHO (2)
R-NHCONHNH₂ (3)
dans lesquelles R et Y sont tels que définis dans l'une quelconque des revendications précédentes.

7. Composition pharmaceutique utilisable dans le traitement des infections à HIV, contenant, comme composants actifs, des précurseurs d'un composé de formule (1) tel que défini dans l'une quelconque des revendications 1 à 5, ces précurseurs étant capables d'auto-assemblage pour former le composé de formule (1), en présence de cellules infectées par le virus.

8. Composition pharmaceutique utilisable dans le traitement d'infections à rétrovirus, contenant, comme composants actifs, des composés de formules :
R-NHCONHN=CH-Y-CHO (2)
et
R-NHCONHNH₂ (3)
dans lesquelles R et Y sont tels que définis dans l'une quelconque des revendications 1 à 5.

9. Composition selon la revendication 8, dans laquelle les composés de formules (2) et (3) sont désignés respectivement par A2 et LY-CH, et répondent aux formules : 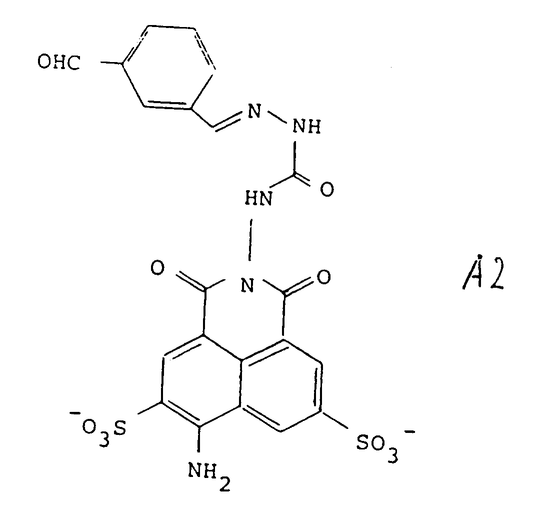 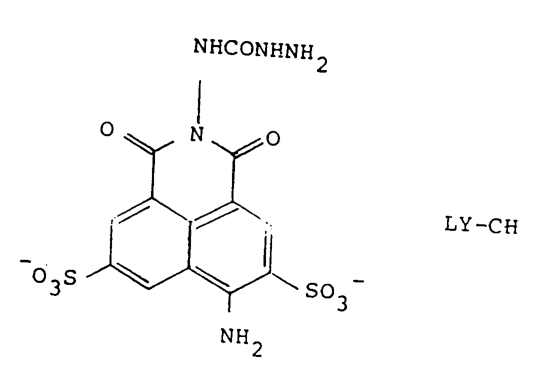 et le composé de formule (1) est H2, tel que défini dans la revendication 5.

10. Combinaison de précurseurs d'un composé de formule (1) comme revendiqué dans l'une quelconque des revendications 1 à 5, qui sont capables d'auto-assemblage pour former le composé de formule (1) en présence de cellules infectées par un virus ou susceptibles d'être infectées par un virus, utilisable dans le traitement d'infections à rétrovirus chez un patient humain.

11. Combinaison de précurseurs selon la revendication 10, de formules (2) et (3), tels que définis dans la revendication 8 ou 9.

12. Utilisation d'un précurseur d'un composé de formule (1), comme revendiqué dans l'une quelconque des revendications 1 à 5, en combinaison avec un autre précurseur d'un composé de formule (1), ces précurseurs étant capables d'auto-assemblage pour former le composé de formule (1) en présence de cellules infectées par un virus ou susceptibles d'être infectées par un virus, dans la fabrication d'un médicament utilisable dans le traitement d'infections à rétrovirus chez un patient humain.

13. Utilisation, selon la revendication 12, d'un précurseur qui est un composé de formule (3)
R-NHCONHNH₂ (3)
en combinaison avec un autre précurseur qui est un composé de formule (2)
R-NHCONHN=CH-Y-CHO (2)
dans lesquelles R et Y sont tels que définis dans l'une quelconque des revendications 1 à 5.

14. Utilisation, selon la revendication 13, de LY-CH en combinaison avec A2, où LY-CH et A2 sont tels que définis dans la revendication 9.

15. Composé utilisable dans le traitement d'infections à rétrovirus chez un patient humain, ce composé répondant à la formule (2)
R-NHCONHN=CH-Y-CHO (2)
utilisable en combinaison avec un autre précurseur qui est un composé de formule (3)
R-NHCONHNH₂ (3)
dans lesquelles R et Y sont tels que définis dans l'une quelconque des revendications 1 à 5.

16. Composé selon la revendication 15 qui est A2, utilisable en combinaison avec le précurseur LY-CH, où A2 et LY-CH sont tels que définis dans la revendication 9.

17. Composé de formule (4) 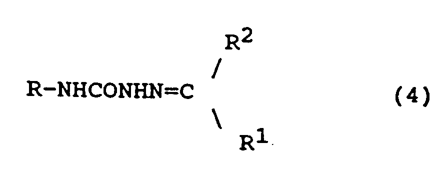 dans laquelle R répond à la formule : 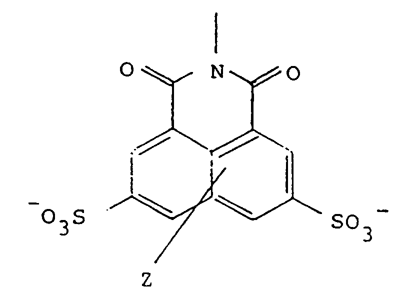 dans laquelle Z représente un ou deux substituants non interférants ;
et dans laquelle
R¹ représente H ou un groupe alkyle en C₁-C₆ ; et
R² représente un groupe alkyle en C₁-C₆ ou aryle en C₆-C₁₀, ou aryle lié en C₆-C₁₀ contenant 2-5 groupes aryle de ce type, lesdits groupes alkyle ou aryle étant non substitués ou substitués par 1 ou 2 substituants non interférants ;
ou un de leurs sels pharmaceutiquement acceptables.

18. Composé selon la revendication 17, dans lequel Z représente un ou deux substituants non interférants, qui sont des groupes alkyle inférieur, alcoxy inférieur, hydroxy, amino, thio, alkylamino, alkylthio ou halogéno ; ou un de leurs sels pharmaceutiquement acceptables.

19. Composé selon la revendication 17 ou 18, dans lequel R² est un groupe alkyle en C₁-C₆ ou aryle en C₆-C₁₀ ou aryle lié en C₆-C₁₀ contenant 2-5 groupes aryle de ce type, lesdits groupes aryle étant non substitués ou substitués par 1 ou 2 substituants non interférants qui sont des groupes alkyle inférieur, alcoxy inférieur, hydroxy, amino, thio, alkylamino, alkylthio, halogéno, carboxy, carbalcoxy ou -CHO ; ou un de leurs sels pharmaceutiquement acceptables.

20. Composé selon la revendication 19, répondant à la formule : 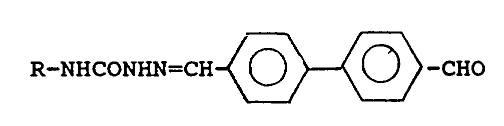 ou un de ses sels pharmaceutiquement acceptables.

21. Composé selon la revendication 20, de formule : 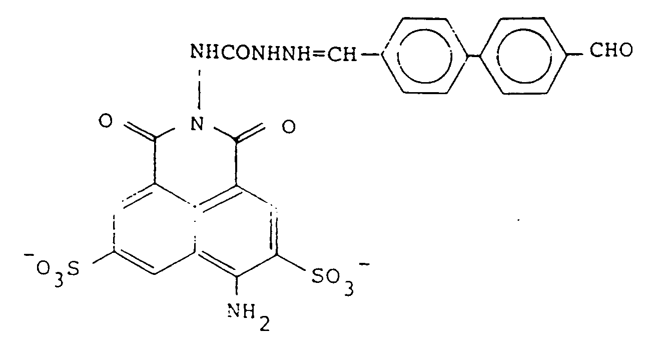 ou un de ses sels pharmaceutiquement acceptables.

22. Composition pharmaceutique utilisable dans la prévention ou le traitement d'infections à rétrovirus, contenant, comme composant actif, une quantité efficace d'un composé selon l'une quelconque des revendications 17 à 21.

23. Procédé de synthèse d'un composé selon l'une quelconque des revendications 17 à 21, ledit procédé comprenant les étapes consistant à mettre en contact un composé de formule (3)
R-NHCONHNH₂ (3)
dans laquelle R est tel que défini dans l'une quelconque des revendications 17 à 21, avec un composé de formule (5) 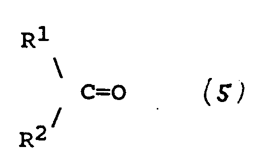 dans laquelle R¹ et R² sont tels que définis dans l'une quelconque des revendications 17 à 21 ; et à récupérer le composé de formule (4) tel que défini à la revendication 17.

24. Composé selon l'une quelconque des revendications 17 à 21, utilisable dans le traitement ou la prévention d'infections à rétrovirus chez un animal.
